(19) Europäisches Patentamt
European Patent Office
Office européen des brevets

(11) Veröffentlichungsnummer: **0 064 657**
**B1**

(12) # EUROPÄISCHE PATENTSCHRIFT

(45) Veröffentlichungstag der Patentschrift: **03.07.85**

(21) Anmeldenummer: **82103512.8**

(22) Anmeldetag: **26.04.82**

(51) Int. Cl.⁴: **C 07 D 263/26**, C 07 D 277/14, C 07 D 277/34, C 07 D 263/58, A 61 K 31/42, A 61 K 31/425

(54) **Ringförmige Sulfenamide, Verfahren zu ihrer Herstellung, sie enthaltende Arzneimittel und deren Herstellung.**

(30) Priorität: **07.05.81 DE 3118129**

(43) Veröffentlichungstag der Anmeldung: **17.11.82 Patentblatt 82/46**

(45) Bekanntmachung des Hinweises auf die Patenterteilung: **03.07.85 Patentblatt 85/27**

(84) Benannte Vertragsstaaten: **AT BE CH DE FR GB IT LI NL SE**

(56) Entgegenhaltungen:
**DE - B - 1 302 649**
**FR - A - 2 409 993**
**GB - A - 835 990**
**GB - A - 927 834**
**GB - A - 955 946**
**US - A - 2 553 775**
**US - A - 2 856 410**

(73) Patentinhaber: **BAYER AG, Konzernverwaltung RP Patentabteilung, D-5090 Leverkusen 1 Bayerwerk (DE)**

(72) Erfinder: **Busse, Wolf-Dieter, Dr., Pahlkestrasse 65, D-5600 Wuppertal 1 (DE)**
Erfinder: **Krauthausen, Edmund, Dr., Franz-Grillparzer-Ring 4, D-5000 Köln 71 (DE)**
Erfinder: **Mardin, Mithat, Dr., Bergerheide 39, D-5600 Wuppertal 1 (DE)**

**EP 0 064 657 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Sulfenamide, ein Verfahren zu ihrer Herstellung, ihre Verwendung als Arzneimittel, insbesondere ihre Verwendung als Lipoxygenasehemmer, diese enthaltende Arzneimittel und deren Herstellung.

Es ist bekannt, dass die durch das Enzym Lipoxygenase gebildeten Metaboliten der Arachidonsäure an der Entstehung von entzündlichen und allergischen Prozessen beteiligt sind [vgl. E.J. Goetzl, Immunology 40, 709 (1980); Ford-Hutchinson et al., J. Pharm. Pharmacol. 32, 517 (1980) und Nature 286, 264 (1980) und Samuelsson, Trends in Pharmacol. Sci., Mai 1980, 227; Borgeat et al., J. Med. Chem. 24, 121 (1981)].

Bekannte Lipoxygenasehemmer wie Nordihydroguaretsäure, 3-Amino-1-(3-trifluormethylphenyl)-pyrazolin, Phenidon und 5,8,11,14-Eikosatetrainsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und zu einer Stimulation des Lipoxygenaseweges, was eine Gastrotoxizität bzw. pro inflammatorische und asthmatische Wirkungen verursacht [vgl. Yen und Kreutner, Agents und Actions, 10, 274 (1980) und Blackwell und Flower, Prostaglandins 16, 417 (1978); und vgl. ebenso Brune et al., J. Pharm. Pharmacol. 33, 127-128 (1981); Higgs et al., Eur. J. Pharmacol. 66, 81-86 (1980) und Piper et al., Prostaglandins 19, 371 (1980)]. Es besteht ein dringendes Bedürfnis nach Verbindungen, die diese unerwünschten Nebenwirkungen nicht besitzen.

Die DE-PS 1 302 649 und die GB-PS 835 990 betreffen Benzoxazolone-(2), die als Fungizide eingesetzt werden können.

Überraschenderweise hemmen die erfindungsgemässen Sulfenamide die Lipoxygenase sehr spezifisch bereits in solchen Konzentrationen, bei denen die Cyclooxygenase nicht beeinflusst wird. Bei Kenntnis des Standes der Technik konnte diese sehr starke und spezifische Wirkung der Sulfenamide nicht erwartet werden. Die erfindungsgemässen, Lipoxygenase hemmenden Sulfenamide sind somit als Arzneimittel bei der Behandlung von entzündlichen und allergischen Prozessen einsetzbar. Sie können insbesondere als Antiphlogistika, Antirheumatika, Antiatherosklerotika, Antiasthmatika, Antiallergika, Antimetastatika und Gastroprotektiva Verwendung finden.

Die erfindungsgemässen Sulfenamide entsprechen der allgemeinen Formel I

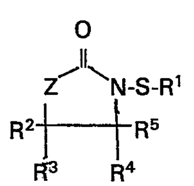

(I)

worin

Z Sauerstoff oder Schwefel bedeutet,

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind, oder $R^1$ für den Rest

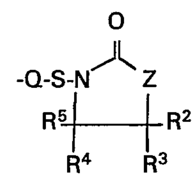

steht, worin Z die oben und $R^2$ bis $R^5$ die unten angegebene Bedeutung besitzen und

Q für Alkylen mit 1 bis 12 C-Atomen, wobei der Alkylenrest durch Sauerstoff, Schwefel oder Stickstoff ein- oder mehrfach unterbrochen sein kann, Cycloalkylen mit 5 bis 12 C-Atomen, Arylen mit 6 bis 10 C-Atomen, Alkylen-Cycloalkylen-Alkylen oder Alkylen-Arylen-Alkylen steht, und

$R^2$ bis $R^5$ unabhängig voneinander für Wasserstoff, gegebenenfalls substituiertes Alkyl mit 1 bis 8 C-Atomen, Cycloalkyl mit 5 bis 8 C-Atomen, Aralkyl mit 7 bis 10 C-Atomen, Aryl mit 6 oder 10 C-Atomen, Alkoxy mit 1 bis 12 C-Atomen, Aralkoxy mit 7 bis 10 C-Atomen, Aryloxy mit 6 oder 10 C-Atomen, Alkylthio mit 1 bis 12 C-Atomen, Benzylthio, Arylthio mit 6 oder 10 C-Atomen und Alkyl-, Benzyl- und/oder Aryl substituiertes Amino stehen, wobei der Alkylrest 1 bis 8 C-Atome und der Arylrest 6 oder 10 C-Atome enthalten kann, oder

$R^2$ zusammen mit $R^4$ eine direkte Bindung oder einen Alkylenrest mit 3 bis 6 C-Atomen bilden oder

$R^2$ und $R^3$ gemeinsam für Alkyliden mit 1 bis 4 C-Atomen, Aralkyliden mit 7 bis 10 C-Atomen, Oxo, Thiono oder substituiertes Imino stehen oder die Reste $R^2$ und $R^3$ zusammen mit dem C-Atom, an das sie gebunden sind, einen Cycloalkylrest mit 5 bis 8 Ringgliedern bilden oder

$R^4$ und $R^5$ oder $R^2$ und $R^3$ gemeinsam für Alkyliden mit 1 bis 4 C-Atomen, Aralkyliden mit 7 bis 10 C-Atomen, Oxo oder substituiertes Imino stehen oder die Reste $R^4$ und $R^5$ zusammen mit dem C-Atom, an das sie gebunden sind, einen Cycloalkylrest mit 5 bis 8 Ringgliedern bilden oder die Reste

$R^2$ bis $R^5$ gemeinsam mit den C-Atomen, an die sie gebunden sind, einen gegebenenfalls substituierten, annellierten Benzoring bilden,

ausgenommen N-(Trichloromethylsulphenyl)- und N-(Dichloro-fluoromethylsulphenyl)-benzoxazolone-(2) bzw. benzthiazolone-(2).

Bevorzugt stehen

$R^1$ für Alkyl mit 1 bis 18 C-Atomen, Cycloalkyl mit 5 bis 8 C-Atomen oder für Aryl mit 6 bis 14 C-Atomen, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl, mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethyl, Trifluormethoxy, Fluor, Chlor, Brom Amino, Alkyl- oder Benzyl-substituiertes Amino, wobei die Alkylreste 1 bis 4 Kohlenstoffatome tragen, substituiert sind, und

$R^2$ bis $R^5$ unabhängig voneinander für Wasserstoff, Alkyl mit 1 bis 4 C-Atomen oder Phenyl, oder

$R^2$ bildet mit $R^4$ eine direkte Bindung, oder

$R^2$ und $R^3$ bzw. $R^4$ und $R^5$ stehen gemeinsam für eine Oxo-Gruppe.

Besonders bevorzugt stehen

Z für Sauerstoff

$R^1$ für einen Arylrest mit 6 oder 10 C-Atomen, der gegebenenfalls mit bis zu 2 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carbalkoxy mit 1 bis 4 Kohlenstoffatomen, Cyano, Trifluormethyl, Trifluormethoxy, Fluor, Chlor, Brom, Alkyl- oder Benzylsubstituiertes Amino, wobei die Alkylreste 1 bis 4 Kohlenstoffatome tragen, substituiert ist, und

$R^2$ bis $R^5$ unabhängig voneinander für Methyl oder Wasserstoff.

Die erfindungsgemässen Verbindungen lassen sich durch Umsetzung von Sulfenhalogeniden der Formel II

$$R^1\text{-S-X} \qquad \text{(II)}$$

worin

$R^1$ die oben angegebene Bedeutung hat und

X für Chlor, Brom oder Jod, vorzugsweise für Chlor steht,

mit cyclischen Verbindungen der allgemeinen Formel III

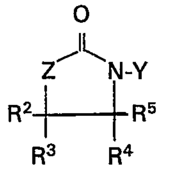

(III)

worin

Z und $R^2$ bis $R^5$ die oben angegebene Bedeutung haben und

Y für Wasserstoff, Trialkylsilyl, ein Metall wie K, Na, Li, Mg, Ca, Ba, Ag, Cu, Zn, Fe, Mn, Pb, Sn oder Al oder einen Ammoniumrest steht

unter Abspaltung von X-Y herstellen.

Für den Fall, dass Y Wasserstoff bedeutet, ist es zweckmässig, die Umsetzung in Gegenwart einer Base durchzuführen. Als Base seien vorzugsweise genannt organische Verbindungen wie Triethylamin, Tributylamin, Benzyldimethylamin, Dimethylanilin, Pyridin oder Chinolin. Die Reaktion wird vorzugsweise in Gegenwart eines aprotischen Lösungsmittels wie z.B. Hexan, Ligroin, Toluol, Chlorbenzol, Chloroform, Tetrachlorkohlenstoff, Dimethylsulfoxid oder Dimethylformamid durchgeführt.

Für den Fall, dass Y nicht für einen Trialkylsilylrest steht, kann es zweckmässig sein, die Herstellung in einem wässrig-organischen Zweiphasenmedium durchzuführen. In diesem Falle können auch anorganische Basen als Halogenwasserstoff-Fänger eingesetzt werden, wie z.B. Alkali- oder Erdalkalihydroxide oder -carbonate.

Die Reaktion kann bei Temperaturen zwischen —80 und +150°C, vorzugsweise zwischen 0 und 50°C, durchgeführt werden.

Die für die Durchführung der Erfindung geeigneten Amine der allgemeinen Formel (III) sind bekannt oder können nach bekannten Methoden hergestellt werden [vgl. R.C. Elderfield (Ed.), Heterocyclic Compounds, B d. 5, S. 396 ff., 405 ff., 711 ff., J. Wiley & Sons, New York 1957; Cook et al., J. Chem. Soc. 1949, 2367].

Die für die Durchführung der Erfindung geeigneten Sulfenhalogenide der allgemeinen Formel (II) sind bekannt oder können ebenfalls nach bekannten Methoden hergestellt werden (vgl. E. Kühle, The Chemistry of the Sulfenic Acids, G. Thieme Verlag, Stuttgart 1973, S. 2-37).

Beispiele erfindungsgemässer Verbindungen sind:

3-Phenylthio-oxazolidin-2-on,
3-(4-Chlorphenylthio)-oxazolidin-2-on,
3-(4-tert.-Butylphenylthio)-oxazolidin-2-on,
3-Pentachlorphenylthio-oxazolidin-2-on,
3-Cyclohexylthio-oxazolidin-2-on,
3-Isopropylthio-oxazolidin-2-on,
3-Naphthylthio-oxazolidin-2-on,
3-(4-methylphenylthio)-oxazolidin-2-on,
3-(3-Trifluormethyl-4-chlorphenylthio)-oxazolidin-2-on,
3-(2-Carbomethoxyphenylthio)-thiazolidin-2-on,
9-Phenylthio-9-aza-8-oxo-7-oxo-spiro-[5,4]-decan,
5-Benzyliden-3-(3,4-dichlorphenylthio)-4-thiono-oxazolidin-2-on,
5,5-Dimethyl-3-tert.-butylthio-oxazolidin-2,4-dion,
5-Isopropyliden-4-phenyl-3-phenylthio-oxazolidin-2-on,
3-Benzylthio-$\Delta^4$-oxazolidin-2-on,
4-Benzyliden-3-(4-ethoxyphenylthio)-oxazolidin-2-on,
3-Phenylthiobenzothiazolin-2-3H-on,
3-(3-Chlor-4-nitrophenylthio)-4,4-methyl-thiazolidin-2-on,
3-(4-Fluorphenylthio)-benzoxazolin-2-on,
3-(4-Chlorphenylthio)-4-phenylthiazolin-2-on,
3-(4-Fluorphenylthio)-oxazolidin-2-on.

Der Nachweis der lipoxygenasehemmenden Eigenschaften der Sulfenamide erfolgt analog der Methode von Bailey et al., J. of Biol. Chem. 255, 5996, (1980) und nach Blackwell und Flower, Prostaglandins 16, 417 (1978). Bei dieser Testmethode wird

der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschliessend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8,10-heptadekatriensäure (HHT) bzw. das Lipoxygenaseprodukt 12-Hydroxy-5,8,11,14-eikosatetraensäure (HETE) unter dem Einfluss der Inhibitoren stellt ein Mass für die Hemmung der Enzyme dar.

Die Lipoxygenasehemmung der erfindungsgemässen Sulfenamide lässt sich an der Hemmung der HETE-Synthese messen. Es zeigt sich, dass die Synthese von $TXB_2$ und von HHT unbeeinflusst bleibt, während der Arachidonsäureumsatz abnimmt. Wie aus der nachfolgenden Tabelle ersichtlich ist, bewirken die Sulfenamide eine signifikante Hemmung der Plättchenlipoxygenase (HETE-Synthese).

*Hemmung der Plättchenlipoxygenasse (HETE-Synthese)*

| Verbindung aus Beispiel Nr. (s.u.) | Minimale effektive Hemmkonzentration (g/ml) (mindestens 50% Hemmung) |
|---|---|
| 2 | $10^{-6}$ |
| 4 | $10^{-7}$ |
| 5 | $3 \cdot 10^{-7}$ |

Die erfindungsgemässen Sulfenamide sind auch in vivo wirksam. Diese Wirkung wird durch die Messung der Hemmung der Leukozytenmigration nach an sich bekannten Methoden nachgewiesen [vgl. Higgs et al., Biochemical Pharmacology 28, 1959, (1979) und Eur. J. Pharmacol. 66, 81 (1980)]. In der nachfolgenden Tabelle 2 sind die Wirkungen einiger beispielhafter Sulfenamide nach lokaler Applikation, durch Einführen eines mit Wirkstoff getränkten Schwämmchens unter die Rückenhaut von Ratten, zusammengefasst.

| Verbindung Nr. | Dosis, lokal (mg/Ratte) | Hemmung der Leukozytenmigration (Kontrolle = 0%) |
|---|---|---|
| 2 | 10 | 55% |
| 4 | 10 | 80% |
| 5 | 10 | 72% |
| 10 | 10 | 53% |

Die antiasthmatische Wirkung der erfindungsgemässen Verbindung kann ebenfalls nach bereits bekannten Methoden nachgewiesen werden [vgl. Samuelson et al., FEBS Letters, 110, 213 (1980) und Yen et al., Agents and Actions 10, 274 (1980)].

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein. d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Ernuss-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh- Milch- und Traubenzucker), Emulgiermittel, wie nichtionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich ausser den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg

Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Die folgenden Beispiele sollen die Erfindung näher erläutern:

*Beispiel 1*

Eine Lösung von 0,6 Mol 2-Propylsulfenchlorid in 200 ml Chlorbenzol tropfte man bei 0 bis 10°C zu 52,2 g (0,6 Mol) Oxazolidin-2-on und 85 g (0,62 Mol) Dimethylbenzylamin in 200 ml Chlorbenzol und 50 ml Dimethylformamid. Man liess 1,5 Stunden bei Raumtemperatur nachrühren, saugte vom ausgefallenen Amin-Hydrochlorid ab, wusch die Mutterlauge mit Wasser, trocknete über Natriumsulfat und entfernte anschliessend das Lösungsmittel im Vakuum bei 40°C. Man erhielt 58 g 3-(2-propylthio)-oxazolidin-2-on als braunes Öl.

$^1$H-NMR ($CDCl_3$): $\delta$ = 1,27 (d, J = 7 Hz, 6H); 3,43 (heptett, J = 7 Hz, 1H) und 3,65-4,6 ppm (m, AA'BB'-System, 4H).

*Beispiel 2*

Analog Beispiel 1 erhielt man aus 0,6 Mol Phenylsulfenchlorid und Oxazolidin-2-on 51,5 g 3-Phenylthio-oxazolidin-2-on als braunes Öl.

$^1$H-NMR ($CDCl_3$): $\delta$ = 3,55-4,5 (m, AA'BB'-System, 4H) und 7,32 ppm (zentriertes m, 5H).

*Beispiel 3*

Analog Beispiel 1 erhält man aus Pentachlorphenylsulfenchlorid und Oxazolidin-2-on 100 g 3-Pentachlorphenylthio-oxazolidin-2-on mit Schmp. 195 bis 197°C.

*Beispiel 4*

Analog Beispiel 1 erhält man aus 0,025 Mol 4-Chlorphenylsulfenchlorid in 100 ml Toluol und 2,2 g (0,025 Mol) Oxazolidin-2-on in 20 ml DMF 5 g 3-(4--Chlorphenylthio)-oxazolidin-2-on als farbloses Pulver mit Schmp. 65-70°C.

*Beispiel 5*

Analog Beispiel 4 erhält man aus 4-tert.-Butylphenylsulfenchlorid und Oxazolidin-2-on 2,7 g 3-(4--tert.-Butylphenylthio)-oxazolidin-2-on als farblose Kristalle mit Schmp. 76-81°C.

*Beispiel 6*

Analog Beispiel 4 erhält man aus 4-Chlorphenylsulfenchlorid und 5-Phenoxymethyloxazolidin-2-on in 33% Ausbeute 3-(4-Chlorphenylthio)-5-phenoxymethyloxazolidin-2-on mit Schmp. 98-101°C

*Beispiel 7*

Analog Beispiel 4 erhält man aus 4-Fluorphenylsulfenchlorid und Oxazolidin-2-on 3-(4-Fluorphenylthio)-oxazolidin-2-on als hellgelbes Öl. $^1$H-NMR- und Massenspektrum stehen im Einklang mit der angegebenen Struktur.

Analog Beispiel 4 tropft man 4-Chlorphenylsulfenchlorid zu den angegebenen Heterocyclen:

*Beispiel 8*

Aus Benzoxazolidin-2-on erhält man 3-(4-Chlorphenylthio)-benzoxazolin-2-on in 62% Ausbeute mit Schmp. 134-137°C.

*Beispiel 9*

Aus 4,4-Dimethyloxazolidin-2-on erhält man 3-(4--Chlorphenylthio-4,4-dimethyloxazolidin-2-on mit Schmp. 124-126°C.

*Beispiel 10*

Aus 4-Phenyl-4-thiazolin-2-on erhält man 3-(4--Chlorphenylthio)-4-phenylthiazolin-2-on in 69% Ausbeute mit Schmp. 205-207°C.

*Beispiel 11*

Aus 5-Phenyloxazolidin-2-on erhält man 3-(4--Chlorphenylthio)-5-phenyloxazolidin-2-on in 80% Ausbeute mit Schmp. 98-99°C.

*Beispiel 12*

Aus Thiazolidin-2,4-dion erhält man 3-(4-Chlorphenylthio)-thiazolidin-2,4-dion in 77% Ausbeute als bräunlichgelbes Öl. Das Massenspektrum steht im Einklang mit der angegebenen Struktur.

*Beispiel 13*

Aus 3a,4,5,6,7,7a-Hexahydrobenzoxazolin-2-on erhält man 3-(4-Chlorphenylthio)-3a,4,5,6,7,7a--hexahydrobenzoxazolin-2-on als zähes gelbes Öl in 92% Ausbeute. Das Massenspektrum steht im Einklang mit der angegebenen Struktur.

**Patentansprüche**

1. Sulfenamide der allgemeinen Formel I

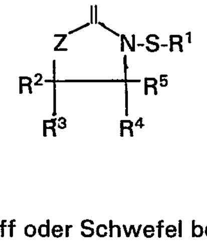

(I)

in welcher

Z Sauerstoff oder Schwefel bedeutet,

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind, oder $R^1$ für den Rest

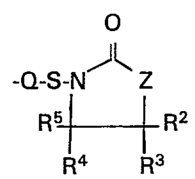

steht, worin Z die oben und $R^2$ bis $R^5$ die unten angegebene Bedeutung besitzen und

Q für Alkylen mit 1 bis 12 C-Atomen, wobei der Alkylenrest durch Sauerstoff, Schwefel oder Stickstoff ein- oder mehrfach unterbrochen sein kann, Cycloalkylen mit 5 bis 12 C-Atomen, Arylen mit 6 bis 10 C-Atomen, Alkylen-Cycloalkylen-Alkylen oder Alkylen-Arylen-Alkylen steht, und

$R^2$ bis $R^5$ unabhängig voneinander für Wasserstoff, gegebenenfalls substituiertes Alkyl mit 1 bis 8 C-Atomen, Cycloalkyl mit 5 bis 8 C-Atomen, Aralkyl mit 7 bis 10 C-Atomen, Aryl mit 6 oder 10 C-Atomen, Alkoxy mit 1 bis 12 C-Atomen, Aralkoxy mit 7 bis 10 C-Atomen, Aryloxy mit 6 oder 10 C-Atomen, Alkylthio mit 1 bis 12 C-Atomen, Benzylthio, Arylthio mit 6 oder 10 C-Atomen und Alkyl-, Benzyl- und/oder Aryl substituiertes Amino stehen, wobei der Alkylrest 1 bis 8 C-Atome und der Arylrest 6 oder 10 C-Atome enthalten kann, oder

$R^2$ zusammen mit $R^4$ eine direkte Bindung oder einen Alkylenrest mit 3 bis 6 C-Atomen bilden oder

$R^2$ und $R^3$ gemeinsam für Alkyliden mit 1 bis 4 C-Atomen, Aralkyliden mit 7 bis 10 C-Atomen, Oxo, Thiono oder substituiertes Imino stehen oder die Reste $R^2$ und $R^3$ zusammen mit dem C-Atom an das sie gebunden sind, einen Cycloalkylrest mit 5 bis 8 Ringgliedern bilden oder

$R^4$ und $R^5$ oder $R^2$ und $R^3$ gemeinsam für Alkyliden mit 1 bis 4 C-Atomen, Aralkyliden mit 7 bis 10 C-Atomen, Oxo oder substituiertes Imino stehen oder die Reste $R^4$ und $R^5$ zusammen mit dem C-Atom, an das sie gebunden sind, einen Cycloalkylrest mit 5 bis 8 Ringgliedern bilden oder die Reste

$R^2$ bis $R^5$ gemeinsam mit den C-Atomen, an die sie gebunden sind, einen gegebenenfalls substituierten, annellierten Benzoring bilden,

ausgenommen N-(Trichloromethylsulphenyl)- und N-(Dichloro-fluoromethylsulphenyl)-benzoxazolone-(2) bzw. benzthiazolone-(2).

2. Sulfenamide der allgemeinen Formel I gemäss Anspruch 1, in welcher

Z für Sauerstoff oder Schwefel,

$R^1$ für Alkyl mit 1 bis 18 C-Atomen, Cycloalkyl mit 5 bis 8 C-Atomen oder für Aryl mit 6 bis 14 C-Atomen, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl, mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethyl, Trifluormethoxy, Fluor, Chlor, Brom, Amino, Alkyl- oder Benzyl-substituiertes Amino, wobei die Alkylreste 1 bis 4 Kohlenstoffatome tragen, substituiert sind, und

$R^2$ bis $R^5$ unabhängig voneinander für Wasserstoff, Alkyl mit 1 bis 4 C-Atomen oder Phenyl, stehen oder

$R^2$ mit $R^4$ eine direkte Bindung bilden oder

$R^2$ und $R^3$ und/oder $R^4$ und $R^5$ gemeinsam für eine Oxo-Gruppe stehen.

3. Sulfenamide der allgemeinen Formel I gemäss Anspruch 1, in welcher

Z für Sauerstoff,

$R^1$ für einen Arylrest mit 6 oder 10 C-Atomen, der gegebenenfalls mit bis zu 2 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carbalkoxy mit 1 bis 4 Kohlenstoffatomen, Cyano, Trifluormethyl, Trifluormethoxy, Fluor, Chlor, Brom, Alkyl- oder Benzyl-substituiertes Amino, wobei die Alkylreste 1 bis 4 Kohlenstoffatome tragen, substituiert ist, und

$R^2$ bis $R^5$ unabhängig voneinander für Methyl oder Wasserstoff stehen.

4. Verfahren zur Herstellung von Sulfenamiden der allgemeinen Formel (I),

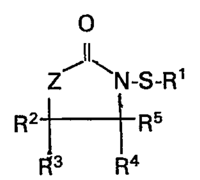

(I)

in welcher

Z Sauerstoff oder Schwefel bedeutet,

$R^1$ für Alkyl mit 1 bis 18 C-Atomen, Aralkyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind, oder $R^1$ für den Rest

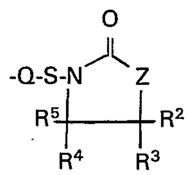

steht, worin Z die oben und $R^2$ bis $R^5$ die unten angegebene Bedeutung besitzen und

Q für Alkylen mit 1 bis 12 C-Atomen, wobei der Alkylenrest durch Sauerstoff, Schwefel oder Stickstoff ein- oder mehrfach unterbrochen sein kann, Cycloalkyl mit 5 bis 12 C-Atomen, Arylen mit 6 bis 10 C-Atomen, Alkylen-Cycloalkylen-Alkylen oder Alkylen-Arylen-Alkylen steht, und

$R^2$ bis $R^5$ unabhängig voneinander für Wasserstoff, gegebenenfalls substituiertes Alkyl mit 1 bis 8 C-Atomen, Cycloalkyl mit 5 bis 8 C-Atomen, Aralkyl mit 7 bis 10 C-Atomen, Aryl mit 6 oder 10 C-Atomen, Alkoxy mit 1 bis 12 C-Atomen, Aralkoxy mit 7 bis 10 C-Atomen, Aryloxy mit 6 oder 10 C-Atomen, Alkylthio mit 1 bis 12 C-Atomen, Benzylthio, Arylthio mit 6 oder 10 C-Atomen und Alkyl-, Benzyl- und/oder Aryl-substituiertes Amino stehen, wobei der Alkylrest 1 bis 8 C-Atome und der Arylrest 6 oder 10 C-Atome enthalten kann, oder

$R^2$ zusammen mit $R^4$ eine direkte Bindung oder einen Alkylenrest mit 3 bis 6 C-Atomen bilden oder

$R^2$ und $R^3$ gemeinsam für Alkyliden mit 1 bis 4 C-Atomen, Aralkyliden mit 7 bis 10 C-Atomen, Oxo, Thiono oder substituiertes Imino stehen oder die Reste $R^2$ und $R^3$ zusammen mit dem C-Atom, an das sie gebunden sind, einen Cycloalkylrest mit 5 bis 8 Ringgliedern bilden oder

$R^4$ und $R^5$ gemeinsam für Alkyliden mit 1 bis 4 C-Atomen, Aralkyliden mit 7 bis 10 C-Atomen, Oxo oder substituiertes Imino stehen oder die Reste $R^4$ und $R^5$ zusammen mit dem C-Atom, an das sie gebunden sind, einen Cycloalkylrest mit 5 bis 8 Ringgliedern bilden,

dadurch gekennzeichnet, dass man Sulfenhalogenide der allgemeinen Formel (II)

$$R^1\text{-S-X} \qquad (II)$$

worin

$R^1$ die oben angegebene Bedeutung hat und

X für Chlor, Brom oder Jod, vorzugsweise für Chlor steht,

mit cyclischen Verbindungen der allgemeinen Formel III

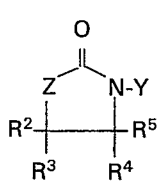

(III)

worin

Z und $R^2$ bis $R^5$ die oben angegebene Bedeutung haben und

Y für Wasserstoff, Trialkylsilyl, ein Metall wie K, Na, Li, Mg, Ca, Ba, Ag, Cu, Zn, Fe, Mn, Pb, Sn oder Al oder einen Ammoniumrest steht,

in Gegenwart von inerten aprotischen Lösungsmitteln und gegebenenfalls in Gegenwart einer Base bei Temperaturen zwischen —80 und +150°C umsetzt.

5. Verfahren gemäss Anspruch 4, dadurch gekennzeichnet, dass man die Reaktion bei Temperaturen zwischen 0 und 50°C durchführt.

6. Verfahren gemäss Anspruch 4 mit der Massgabe, dass Y nicht für einen Trialkylsilylrest steht, dadurch gekennzeichnet, dass man die Reaktion in einem wässrig-organischen Zweiphasenmedium durchführt und für den Fall dass Y für Wasserstoff steht, zusätzlich organische oder anorganische Basen als Halogenwasserstoff-Fänger einsetzt.

7. Arzneimittel, enthaltend mindestens eine Verbindung der allgemeinen Formel (I) gemäss Anspruch 1.

8. Verfahren zur Herstellung von Arzneimitteln nach Anspruch 7, dadurch gekennzeichnet, dass man Sulfenamide der allgemeinen Formel (I) gemäss Anspruch 1 gegebenenfalls unter Verwendung von Hilfs- und Trägerstoffen in eine geeignete Applikationsform überführt.

**Claims**

1. Sulphenamides of the general formula I

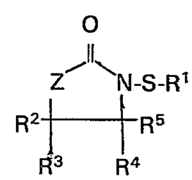

(I)

in which

Z denotes oxygen or sulphur,

$R^1$ represents alkyl with 1 to 18 C atoms, alkenyl or alkinyl with 2 to 12 C atoms, aralkyl with 7 to 11 C atoms, cycloalkyl with 5 to 8 C atoms or aryl with 6 to 14 C atoms, these radicals being optionally substituted by up to 5 identical or different substituents from the group comprising alkoxy, alkyl, aralkyl, cycloalkyl, aryl, aryloxy, arylthio, alkylthio, carboxyl, carbalkoxy, cyano, carbamoyl, sulphonyl, halogenoalkyl, halogenoalkoxy, halogen, amino and substituted amino, or $R^1$ represents the radical

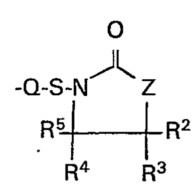

wherein

Z has the meaning given above and

$R^2$ to $R^5$ have the meaning given below, and

Q represents alkylene with 1 to 12 C atoms, it being possible for the alkylene radical to be interrupted by one or more oxygen, sulphur or nitrogen atoms, cyloalkylene with 5 to 12 C atoms, arylene with 6 to 10 C atoms, alkylene-cycloalkylene-alkylene or alkylene-arylene-alkylene, and

$R^2$ to $R^5$ independently of one another represent hydrogen, optionally substituted alkyl with 1 to 8 C atoms, cycloalkyl with 5 to 8 C atoms, aralkyl with 7 to 10 C atoms, aryl with 6 or 10 C atoms, alkoxy with 1 to 12 C atoms, aralkoxy with 7 to 10 C atoms, aryloxy with 6 or 10 C atoms, alkylthio with 1 to 12 C atoms, benzylthio, arylthio with 6 or 10 C atoms and alkyl-, benzyl- and/or aryl-substituted amino, it being possible for the alkyl radical to contain 1 to 8 C atoms and the aryl radical 6 or 10 C atoms, or

$R^2$, together with $R^4$, forms a direct bond or an alkylene radical with 3 to 6 C atoms or

$R^2$ and $R^3$ together represent alkylidene with 1 to 4 C atoms, aralkylidene with 7 to 10 C atoms, oxo, thiono or substituted imino or the radicals $R^2$ and $R^3$, together with the C atom to which they are bonded, form a cycloalkyl radical with 5 to 8 ring members or

$R^4$ and $R^5$ or $R^2$ and $R^3$ together represent alkylidene with 1 to 4 C atoms, aralkylidene with 7 to 10 C atoms, oxo or substituted imino or the radicals $R^4$ and $R^5$, together with the C atom to which they are bonded, form a cycloalkyl radical with 5 to 8 ring members, or the radicals

$R^2$ to $R^5$, together with the C atoms to which they are bonded, form an optionally substituted, fused-on benzene ring,

with the exception of N-(trichloromethylsulphenyl)- and N-(dichlorofluoromethylsulphenyl)-benzoxazol-2-one and benzothiazol-2-one.

2. Sulphenamides of the general formula I according to claim 1, in which

Z represents oxygen or sulphur,

$R^1$ represents alkyl with 1 to 18 C atoms, cycloalkyl with 5 to 8 C atoms or aryl with 6 to 14 C atoms, these radicals being optionally substituted by up to 5 identical or different substituents from the group comprising alkyl and alkoxy with in each case 1 to 4 carbon atoms, aralkyl with 7 to 10 carbon atoms, cycloalkyl with 5 to 7 carbon atoms, phenyl, phenoxy, phenylthio, alkylthio with 1 to 4 carbon atoms, carboxyl, carbalkoxy with 1 to 4 carbon atoms, cyano, trifluoromethyl, trifluoromethoxy, fluorine, chlorine, bromine, amino, alkyl-substituted amino and benzylsubstituted amino, the alkyl radicals carrying 1 to 4 carbon atoms, and

$R^2$ to $R^5$ independently of one another represent hydrogen, alkyl with 1 to 4 C atoms or phenyl or

$R^2$ forms a direct bond with $R^4$ or

$R^2$ and $R^3$ and/or $R^4$ and $R^5$ together represent an oxo group.

3. Sulphenamides of the general formula I according to claim 1, in which

Z represents oxygen,

$R^1$ represents an aryl radical with 6 or 10 C atoms which is optionally substituted by up to 2 identical or different substituents from the group comprising alkyl and alkoxy with in each case 1 to 4 carbon atoms, aralkyl with 7 to 10 carbon atoms, cycloalkyl with 5 to 7 carbon atoms, phenyl, phenoxy, phenylthio, alkylthio with 1 to 4 carbon atoms, carbalkoxy with 1 to 4 carbon atoms, cyano, trifluoromethyl, trifluoromethoxy, fluorine, chlorine, bromine, alkyl-substituted amino and benzyl-substituted amino, the alkyl radicals carrying 1 to 4 carbon atoms, and

$R^2$ to $R^5$ independently of one another represent methyl or hydrogen.

4. Process for the preparation of sulphenamides of the general formula (I)

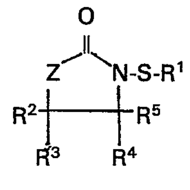

(I)

in which

Z denotes oxygen or sulphur,

$R^1$ represents alkyl with 1 to 18 C atoms, aralkyl or alkinyl with 2 to 12 C atoms, aralkyl with 7 to 11 C atoms, cycloalkyl with 5 to 8 C atoms or aryl with 6 to 14 C atoms, these radicals being optionally substituted by up to 5 identical or different substituents from the group comprising alkoxy, alkyl, aralkyl, cycloalkyl, aryl, aryloxy, arylthio, alkylthio, carboxyl, carbalkoxy, cyano, carbamoyl, sulphonyl, halogenoalkyl, halogenoalkoxy, halogen, amino and substituted amino, or $R^1$ represents the radical

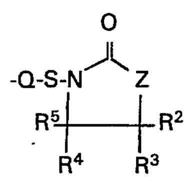

wherein

Z has the meaning given above and

$R^2$ to $R^5$ have the meaning given below, and

Q represents alkylene with 1 to 12 C atoms, it being possible for the alkylene radical to be interrupted by one or more oxygen, sulphur or nitrogen atoms, cyloalkylene with 5 to 12 C atoms, arylene with 6 to 10 C atoms, alkylene-cycloalkylene-alkylene or alkylene-arylene-alkylene, and

$R^2$ to $R^5$ independently of one another represent hydrogen, optionally substituted alkyl with 1 to 8 C atoms, cycloalkyl with 5 to 8 C atoms, aralkyl with 7 to 10 C atoms, aryl with 6 or 10 C atoms, alkoxy with 1 to 12 C atoms, aralkoxy with 7 to 10 C atoms, aryloxy with 6 or 10 C atoms, alkylthio with 1 to 12 C atoms, benzylthio, arylthio with 6 or 10 C atoms and alkyl-, benzyl- and/or aryl-substituted amino, it being possible for the alkyl radical to contain 1 to 8 C atoms and the aryl radical 6 or 10 C atoms, or

$R^2$, together with $R^4$, forms a direct bond or an alkylene radical with 3 to 6 C atoms or

$R^2$ and $R^3$ together represent alkylidene with 1 to 4 C atoms, aralkylidene with 7 to 10 C atoms, oxo, thiono or substituted imino or the radicals $R^2$ and $R^3$, together with the C atom to which they are bonded, form a cycloalkyl radical with 5 to 8 ring members or

$R^4$ and $R^5$ represent together alkylidene with 1 to 4 C atoms, aralkylidene with 7 to 10 C atoms, oxo or substituted imino or the radicals $R^4$ and $R^5$, together with the C atom to which they are bonded, form a cycloalkyl radical with 5 to 8 ring members,

characterised in that sulphenyl halides of the general formula (II)

$$R^1\text{-S-X} \qquad \text{(II)}$$

wherein

$R^1$ has the abovementioned meaning and

X represents chlorine, bromine or iodine, preferably chlorine, are reacted with cyclic compounds of the general formule (III)

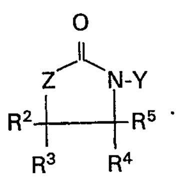

(III)

wherein

Z and $R^2$ to $R^5$ have the abovementioned meaning and

Y represents hydrogen, trialkylsilyl, a metal such as K, Na, Li, Mg, Ca, Ba, Ag, Cu, Zn, Fe, Mn, Pb, Sn or Al or an ammonium radical,

in the presence of inert aprotic solvents and, if appropriate, in the presence of a base at temperatures between —80 and +150°C.

5. Process according to claim 4 characterised in that the reaction is carried out at temperatures between 0 and 50°C.

6. Process according to claim 4, with the proviso that Y does not represent a trialkylsilyl radical, characterised in that the reaction is carried out in an aqueous-organic two-phase medium and in the case where Y represents hydrogen organic or inorganic bases are additionally employed as hydrogen halide removers.

7. Medicaments containing at least one compound of the general formula (I) according to claim 1.

8. Process for the preparation of medicaments according to claim 7, characterised in that sulphenamides of the general formula (I) according to claim 1 are converted into a suitable form of administration, if appropriate using auxiliaries and excipients.

**Revendications**

1. Sulfénamides de formule générale I

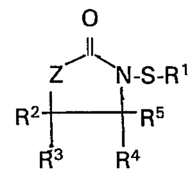

(I)

dans laquelle

Z représente l'oxygène ou le soufre,

$R^1$ représente un groupe alkyle en $C_1$-$C_{18}$, alcényle ou alcynyle en $C_2$-$C_{12}$, aralkyle en $C_7$-$C_{11}$, cycloalkyle en $C_5$-$C_8$ ou aryle en $C_6$-$C_{14}$, ces groupes pouvant le cas échéant porter jusqu'à 5 substituants identiques ou différents choisis parmi les groupes alcoxy, alkyle, aralkyle, cycloalkyle, aryle, aryloxy, arylthio, alkylthio, carboxy, carbalcoxy, cyano, carbamoyle, sulfonyle, halogénoalkyle, halogénoalcoxy, les halogènes, les groupes amino ou amino substités, ou bien $R^1$ représente le reste

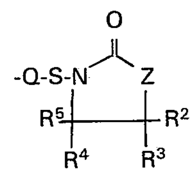

dans lequel

Z a la signification indiquée ci-dessus et $R^2$ à $R^5$ les significations indiquées ci-dessous, et

Q représente un groupe alkylène en $C_1$-$C_{12}$, le groupe alkylène pouvant être interrompu une ou plusieurs fois par l'oxygène, le soufre ou l'azote, un groupe cycloalkylène en $C_5$-$C_{12}$, arylène en $C_6$-$C_{10}$, alkylène-cycloalkylène-alkylène, ou alkylène-arylène-alkylène, et

$R^2$ à $R^5$ représentent, indépendamment les uns des autres, l'hydrogène, un groupe alkyle en $C_1$-$C_8$ éventuellement substitué, cycloalkyle en $C_5$-$C_8$, aralkyle en $C_7$-$C_{10}$, aryle en $C_6$ ou $C_{10}$, alcoxy en $C_1$-$C_{12}$, aralcoxy en $C_7$-$C_{10}$, aryloxy en $C_6$ ou $C_{10}$, alkylthio en $C_1$-$C_{12}$, benzylthio, arylthio en $C_6$ ou $C_{10}$ et amino à substituants alkyle, benzyle et/ou aryle, le groupe alkyle pouvant contenir 1 à 8 atomes de carbone et le groupe aryle 6 ou 10 atomes de carbone, ou bien

$R^2$ représente avec $R^4$ une liaison directe ou un groupe alkylène en $C_3$-$C_6$, ou bien

$R^2$ et $R^3$ représentent ensemble un groupe alkylidène en $C_1$-$C_4$, aralkylidène en $C_7$-$C_{10}$, oxo, thiono ou imino substitué ou bien $R^2$ et $R^3$ forment ensemble et avec l'atome de carbone auquel ils sont reliés un groupe cycloalkyle contenant 5 à 8 chaînons cycliques, ou bien

$R^4$ et $R^5$ ou $R^2$ et $R^3$ représentent ensemble un groupe alkylidène en $C_1$-$C_4$, aralkylidène en $C_7$-$C_{10}$, oxo ou imino substitué ou bien $R^4$ et $R^5$ forment ensemble et avec l'atome de carbone auquel ils sont reliés un grupe cycloalkyle contenant 5 à 8 chaînons cycliques, ou bien

$R^2$ à $R^5$ forment ensemble et avec l'atome de carbone auquel ils sont reliés un cycle benzénique condensé éventuellement substitué,

à l'exception des N-(trichlorométhylsulfényl)- et N-(dichlorofluorométhylsulfényl)-benzoxazolones-2 et -benzothiazolones-2.

2. Sulfénamides de formule générale I selon la revendication 1, dans lesquels:

Z représente l'oxygène ou le soufre,

$R^1$ représente un groupe alkyle en $C_1$-$C_{18}$, cycloalkyle en $C_5$-$C_8$ ou aryle en $C_6$-$C_{14}$, ces groupes pouvant le cas échéant porter jusqu'à 5 substituants identiques ou différents choisis parmi les groupes alkyle, alkoxy contenant chacun 1 à 4 atomes de carbone, aralkyle en $C_7$-$C_{10}$, cycloalkyle en $C_5$-$C_7$, phényle, phénoxy, phénylthio, alkylthio en $C_1$-$C_4$, carboxy, carbalcoxy en $C_1$-$C_4$, cyano, trifluorométhyle, trifluorométhoxy, le fluor, le chlore, le brome, un groupe amino, un groupe amino à substituants alkyle ou benzyle, les groupes alkyle contenant de 1 à 4 atomes de carbone, et

$R^2$ à $R^5$ représentent, indépendamment les uns des autres, l'hydrogène, un groupe alkyle en $C_1$-$C_4$ ou phényle, ou bien

$R^2$ forme avec $R^4$ une liaison directe, ou bien

$R^2$ et $R^3$ et/ou $R^4$ et $R^5$ représentent ensemble un groupe oxo.

3. Sulfénamides de formule générale I selon la revendication 1, dans lesquels

Z représente l'oxygène,

$R^1$ représente un groupe alkyle en $C_6$ ou $C_{10}$ qui peut porter le cas échéant jusqu'à deux substituants identiques ou différents pris parmi les groupes alkyle, alcoxy contenant chacun 1 à 4 atomes de carbone, aralkyle en $C_7$-$C_{10}$, cycloalkyle en $C_5$-$C_7$, phényle, phénoxy, phénylthio, alkylthio en $C_1$-$C_4$, carbalcoxy en $C_1$-$C_4$, cyano, trifluorométhyle, trifluorométhoxy, le fluor, le chlore, le brome, des groupes amino à substituants alkyle ou benzyle, les groupes alkyle contenant 1 à 4 atomes de carbone, et

$R^2$ à $R^5$ représentent indépendamment les uns des autres, des groupes méthyle ou l'hydrogène.

4. Procédé de préparation des sulfénamides de formule générale I

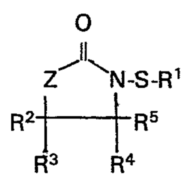

(I)

dans laquelle

Z représente l'oxygène ou le soufre,

$R^1$ représente un groupe alkyle en $C_1$-$C_{18}$, aralkyle ou alcynyle en $C_2$-$C_{12}$, aralkyle en $C_7$-$C_{11}$, cycloalkyle en $C_5$-$C_8$ ou aryle en $C_6$-$C_{14}$, ces groupes pouvant le cas échéant porter jusqu'à 5 substituants identiques ou différents choisis parmi les groupes alcoxy, alkyle, aralkyle, cycloalkyle, aryle, aryloxy, arylthio, alkylthio, carboxy, carbalcoxy, cyano, carbamoyle, sulfonyle, halogénoalkyle, halogénoalcoxy, les halogènes, les groupes amino ou amino substités, ou bien $R^1$ représente le groupe

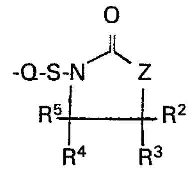

dans lequel

Z a les significations indiquées ci-dessus et $R^2$ à $R^5$ les significations indiquées ci-dessous, et

Q représente un groupe alkylène en $C_1$-$C_{12}$, le groupe alkylène pouvant être interrompu une ou plusieurs fois par l'oxygène, le soufre ou l'azote, un groupe cycloalkylène en $C_5$-$C_{12}$, arylène en $C_6$-$C_{10}$, alkylène-cycloalkylène-alkylène, ou alkylène-arylène-alkylène, et

$R^2$ à $R^5$ représentent, indépendamment les uns des autres, l'hydrogène, un groupe alkyle en $C_1$-$C_8$ éventuellement substitué, cycloalkyle en $C_5$-$C_8$, aralkyle en $C_7$-$C_{10}$, aryle en $C_6$ ou $C_{10}$, alcoxy en $C_1$-$C_{12}$, aralcoxy en $C_7$-$C_{10}$, aryloxy en $C_6$ ou $C_{10}$, alkylthio en $C_1$-$C_{12}$, benzylthio, arylthio en $C_6$ ou $C_{10}$ et amino à substituants alkyle, benzyle et/ou aryle, le groupe alkyle pouvant contenir 1 à 8 atomes de carbone et le groupe aryle 6 ou 10 atomes de carbone, ou bien

$R^2$ représente avec $R^4$ une liaison directe ou un groupe alkylène en $C_3$-$C_6$, ou bien

$R^2$ et $R^3$ représentent ensemble un groupe alkylidène en $C_1$-$C_4$, aralkylidène en $C_7$-$C_{10}$, oxo, thiono ou imino substitué ou bien les symboles $R^2$ et $R^3$ représentent ensemble et avec l'atome de carbone auquel ils sont reliés un groupe cycloalkyle contenant 5 à 8 chaînons cycliques, ou bien

$R^4$ et $R^5$ représentent ensemble un groupe alkylidène en $C_1$-$C_4$, aralkylidène en $C_7$-$C_{10}$, oxo ou imino substitué ou bien $R^4$ et $R^5$ représentent ensemble et avec l'atome de carbone auquel ils sont reliés un groupe cycloalkyle contenant 5 à 8 chaînons cycliques,

ce procédé se caractérisant en ce que l'on fait réagir des halogénures de sulfényle de formule générale II

$$R^1\text{-S-X} \qquad (II)$$

dans laquelle

$R^1$ a les significations indiquées ci-dessus, et

X représente le chlore, le brome ou l'iode, de préférence le chlore,

avec des composés cycliques de formule générale III

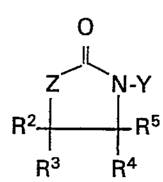

(III)

dans laquelle

Z et $R^2$ à $R^5$ ont les significations indiquées ci-dessus, et

Y représente l'hydrogène, un groupe trialkylsilyle, un métal tel que K, Na, Li, Mg, Ca, Ba, Ag, Cu, Zn, Fe, Mn, Pb, Sn ou Al ou un reste ammonium,

en présence de solvants aprotoniques inertes et le cas échéant en présence d'une base, à des températures allant de —80 à +150°C.

5. Procédé selon la revendication 4, caractérisé en ce que l'on effectue la réaction à des températures de 0 à 50°C.

6. Procédé selon la revendication 4, étant spécifié que Y ne représente pas un groupe trialkylsilyle, caractérisé en ce que l'on effectue la réaction dans un milieu hydro-organique à deux phases et, dans le cas où Y représente l'hydrogène, en utilisant en outre des bases organiques ou minérales en tant que capteurs d'hydracides halogénés.

7. Médicaments contenant au moins un composé de formule générale I selon la revendication 1.

8. Procédé de préparation des médicaments selon la revendication 7, caractérisé en ce que l'on met les sulfénamides de formule générale I selon la revendication 1 sous des formes d'administration appropriées avec utilisation éventuelle de produits auxiliaires et de véhicules.